# EUROPEAN PATENT APPLICATION

(11) **EP 0 950 414 A1**
(43) Date of publication of application: **20.10.1999**
(21) Application number: 98106404.1
(22) Date of filing: 08.04.1998
(51) Int. Cl.: A61K 38/18, A61K 48/00

(54) **Use of a combination of an osteoinductive protein and a dorsalizing factor for cartilage induction**

(71) Applicant: Roche Diagnostics GmbH, 68305 Mannheim (DE)
(72) Inventor: The designation of the inventor has not yet been filed

(57) **Abstract**

A composition of an osteoinductive protein and a dorsalizing factor at a ratio of 10:1 to 1:10 is a useful pharmaceutical agent for chondrogenesis.

## Description

The present invention relates to a method and a composition for the induction of chondrocytes from mesenchymal stem cells using a combination of an osteoinductive protein with a dorsalizing factor.

Osteoinductive proteins are proteins which induce the differentiation of mesenchymal stem cells towards chondrocytes and osteocytes and are, for example, hedgehog proteins (Sonic (Shh), Indian (Ihh), Desert (Dhh); Kinto et al., FEBS Letters 404 (1997) 319-323), or bone morphogenetic proteins.

Bone morphogenetic proteins (BMPs) are molecules which are responsible for the formation of bone, cartilage, tendon, and other tissues present in bone. The unique inductive activities of these proteins, along with their presence in bone, suggest that they are important regulators of bone repair processes and may be involved in the normal maintenance of bone tissue. Many such proteins are known which can be divided into several sub-families (Reddi, A.H., Cytokine & Growth Factor Reviews 8 (1997) 11-20). Such BMPs are, for example, BMP-1 to BMP-14 and GDF-1 to GDF-14.

BMPs are pleiotropic regulators and hence regulate the multistep sequential cascade in bone and cartilage formation such as chemotaxis, mitosis and differentiation. Especially, BMP-2, BMP-4, BMP-5, BMP-7 initiate chondrogenesis and osteogenesis.

However, BMP-2, BMP-4, BMP-5 and BMP-7 are not able to induce chondrogenesis alone without concomitant induction of osteogenesis. Thus the pharmaceutical application of BMP-2, BMP-4, BMP-5 and BMP-7 in connection with endochondral bone formation will always result in chondrocyte and osteocyte induction. In connection with the repair of cartilage tissue, however, it would be advantageous to achieve initiation of chondrogenesis without concomitant induction of osteogenesis.

Bone morphogenetic protein antagonists are substances which inhibit the osteogenic properties of BMPs and are, for example, noggin, chordin, or follistatin.

Noggin is a growth factor and a bone morphogenetic protein (BMP) antagonist which is described, e.g., in U.S. Patent No. 5,670,481. Noggin is expressed in the Spemann organizer and seems to be a mediator of the effects of the Spemann organizer, namely neural induction and dorsalization of the mesoderm. Since noggin is expressed in the notochord and head mesoderm, it seems that noggin influences either the dorsal-ventral pattern or anterior-posterior pattern of the neural plate. Since noggin is expressed in the branchial arch neural crest, it seems that noggin influences whether neural crest cells deposit cartilage and also influences later branchial arch growth and remodelling. Noggin is expressed in the tail fin neural crest, and since neural crest is required for growth of the fin, noggin may act as growth factor for epidermis or mesenchyme.

Noggin can bind BMP-4 with high affinity (Piccollo et al., Cell 86 (1996) 12141-12145) and is an antagonist to BMPs (Re'em-Kalma et al., Proc. Natl. Acad. Sci. USA 92 (1995) 12141-12145; Sasai et al., Nature 376 (1995) 333-336; Fainsod et al., Mechanisms of Development 63 (1997) 39-50).

Chordin is a secreted polypeptide and a potent dorsalizing and neural inducing factor (U.S. Patent No. 5,679,783; Sasai et al., Cell 79 (1994) 779-790). Chordin is expressed in the Spemann organizer. Chordin overexpression induces notochord and neural tissue, but not mesoderm. However, chordin can modify mesoderm specification. Chordin expression overlaps with noggin and can act similarly to noggin. Chordin acts antagonistically to BMP-4 (Fainsod et al., EMBO 13 (1994) 5015-5025) and can bind with BMP-4 with high affinity (Piccollo et al., Cell 86 (1996) 589-598).

Follistatin is a secreted polypeptide and is expressed in the Spemann organizer and acts as dorsalizing and neural inducing factor, originally described as binding protein of activin and inhibiting activin function (Nakamura et al., Science 247 (1990) 836-838; Hemmati-Brivanlou et al., Cell 79 (1994) 169-179). Follistatin can also bind BMPs (Fainsod et al., Mechanisms of Development 63 (1997) 39-50).

In the case of cartilage defects, only limited success has been achieved. Standard procedures are shaving of fibrillated articular cartilage, debridement, spongiolaziation, abrasion chondroplasty, osteotomies, microfracture and cartilage grafting techniques such as perichondral autografts, periostal autografts. A simple and effective treatment is still missing (see review by Heath and Margari, Biotechnology and Bioengineering 50 (1996) 430-437). Novel therapies could involve chondrocyte transplantation, growth factor therapy, cell therapy (U.S. Patent No. 5,486,359), gene therapy, new biomaterials. Currently, these are not yet available.

The invention provides a method for cartilage repair, using a composition of an osteogenic factor and a bone morphogenetic protein antagonist.

It was surprisingly found that osteoinductive (osteogenic) proteins, preferably the bone morphogenetic proteins 2, 4, 5 and 7 or hedgehog proteins are not completely inhibited in vivo by BMP antagonists such as noggin, chordin and follistatin, when present at a ratio of 10 : 1 to 1 : 10 (BMP: noggin). In this, osteogenesis initiation is however inhibited to a large extent whilst chondrogenesis proceeds largely unaffected. For an effect according to the present invention it is essential that the BMP antagonist exhibits a high binding affinity for BMP-2, BMP-4, BMP-5 and BMP-7.

By "osteoinductive protein" is preferably understood an osteogenic protein which induces BMP-dependent osteogenesis based on mesenchymal stem cells. BMPs are thereby upregulated, and this ultimately leads to the formation of chondrocytes. Such an induction of BMP-dependent osteogenesis can be achieved, for instance, through the BMPs themselves or through substances inducing the expression of BMPs in cells, e.g., hedgehog proteins.

A substance's ability to induce BMP-dependent osteogenesis can be tested in a simple manner. For this purpose, for example, mesenchymal cells, e.g., C3H10T1/2 cells, are cultured with and without the potential osteoinductive factor. Controls and treated cells are measured for osteocalcin and alkaline phosphatase activity. Osteocalcin can be measured by commercially available ELISA, e.g., from Dako Co. Alkaline phosphatase can be measured photometrically using a suitable colorimetric substrate, e.g., p-nitrophenyl phosphate. Increased activity of osteocalcin and/or alkaline phosphatase is scored as osteoinduction. Alternatively, upregulation of osteocalcin and alkaline phosphatase is measured by RT-PCR using suitable primers for osteocalcin and alkaline phosphatase.

By a "dorsalizing factor" according to the invention is understood a substance, preferably a protein, which like a bone morphogenetic protein antagonist inhibits the osteoinductive property of BMPs, preferably of BMP-2 or hedgehog. Such an inhibition can be determined, for example, in a bone marrow stromal cell line assay (Zimmermann et al., Cell 86 (1996) 599-606). A dorsalizing factor has the property of promoting the formation of dorsal structures in Xenopus. The dorsalizing activity can be determined by means of a Xenopus animal cap assay. The Xenopus animal cap assay (Ruiz i Altaba, Essential Developmental Biology, A Practical Approach, IRL Press, 1993, IRL Press, pp. 147-152; Lamb et al., Science 262 (1993) 713-718) is used to determine neural induction. After treatment animal caps are stained for the presence of neural markers, e.g., NCAM, and the absence of mesodermal markers, e.g., muscle actin. Such factors are, for example, noggin, chordin, and follistatin (Fainsod et al., Mechanisms of Development 63 (1997) 39-50). BMP antagonists are described, for example, by Re'em-Kalma et al., Proc. Natl. Acad. Sci. USA 92 (1995) 12141-12145; Sasai et al., Nature 376 (1995) 333-336; and Fainsod et al., Mechanisms of Development 63 (1997) 39-50.

In a further preferred embodiment of the invention, the osteogenic protein and the dorsalizing factor can be introduced in the cells via gene therapy. For this method the genes coding for the osteogenic protein and the dorsalizing factor can be introduced in one vector, preferably under the control of the same promoter, or in separate vectors. For an efficient expression of the osteogenic factor and the dorsalizing factor, it is necessary to use strong promoters in the vectors. Such promoters are, e.g., PGK or CMV promoters. Preferably, the expression vector consists of such a strong promoter, the full-length mRNA of the chosen gene, e.g., BMP-2, BMP-4, BMP-5, BMP-7, Shh, Ihh, or Dhh, noggin, chordin, or follistatin, an artificial intron and a poly-A-site. For application, DNA is either lyophilized to collagen sponges or applied with any other suitable carrier, preferably hyaluronic acid or alginate.

The pharmaceutical formulation according to the invention may also include an appropriate matrix, for instance, for delivery and/or support of the composition and/or providing a surface for cartilage formation. The matrix may provide slow release of the osteoinductive protein and the bone morphogenetic protein antagonist. Preferably, the composition includes a matrix which is biocompatible and/or biodegradable. Potential matrices for the compositions contain, for example, hyaluronic acid, alginate, calcium sulfate, tricalcium phosphate, hydroxylapatite, polylactic acid, polyanhydrides, or collagen.

The dosage regimen will be determined by the attending physician, considering various facts which modify the action of the formulation of the invention. Factors which may modify the action of the formulation include the amount of cartilage desired to be formed, the site of application, the condition of the damage, the patient's age, sex and diet, the severity of any infection, time of administration, and other clinical factors. The dosage may vary with the type of the matrix used in the reconstitution of cartilage.

A further object of the invention is a method for manufacturing a pharmaceutical composition containing a bone morphogenetic protein and a bone morphogenetic protein antagonist at a ratio of 10:1 to 1:10 by combining said protein and said antagonist, and the use of such a pharmaceutical composition for chondrogenesis in vivo, especially for the treatment of a patient who suffers from cartilage defects and hence is in need of cartilage repair.

The following examples and references are provided to aid the understanding of the present invention, the true scope of which is set forth in the appended claims. It is understood that modifications can be made in the procedures set forth without departing from the spirit of the invention.

### Example 1

### Micromass mouse limb assay

Limb buds are isolated from E11 mouse embryos (NMRI) using microdissection scissors and watchmaker's forceps under sterile conditions. A cell suspension is obtained by rinsing and incubating in calcium- and magnesium-free saline, then incubation for 20 min in 1% trypsin solution at 37°C, followed by disaggregation by repeated aspiration with a 1 ml pipette. The cells are adjusted to 2 x 10⁷ per ml in Ham's medium and five 10 µl aliquots are placed in sterile Petri dishes. After 2 h of culture, 2 ml of Ham's medium is added, alone or Ham's medium with test compound, consisting of (a) BMP antagonist noggin, chordin or follistatin alone, (b) BMP-2, BMP-4, BMP-5, BMP-7 or Shh, and (c) combinations of BMP-2, BMP-4, BMP-5, BMP-7 or Shh and antagonist in varying doses from less than 1 ng/ml to about 1 µg/ml. The medium is not changed during the five day culture period. Then the cultures are fixed and stained with Alcian Blue or Crystal-Violet, washed in tap water and dried (Steele and Copping, Postimplantation Mammalian Embryos, A Practical Approach, IRL Press 1990, 229-232). Differentiation is assessed after Alcian Blue staining for chondrogenesis, Crystal-Violet for fibroblast-like cells. In addition, quantitative RT-PCR analyses are performed for expression of cartilage (e.g., collagen II, MIA, collagen X) and bone markers (e.g., osteocalcin, alkaline phosphatase, osteopontin).

### Example 2

### Chondrocyte culture

For articular chondrocytes, isolated from 2-month-old mice (NMRI), the tibial plateau is surgically removed from the knee joint and the articular tissue is carefully freed of contaminating adhering tissues, including the fibrocartilage layer facing the synovial cavity. The tissue is minced with a surgical blade, incubated for 16 h in 0.25% trypsin and 0.1% crude collagenase mixture in Hank's buffered saline solution. Cells are grown for nine days in serum-free high glucose Dulbecco's modified Eagle's medium (DMEM), as monolayer or as agarose cultures (three-dimensional matrix) (d'Angelo and Pacifici, J. Bone and Min. Res. 12 (1997) 1368-1377). The cells are treated with (a) BMP antagonist alone, (b) inducing or modifying factor alone, and (c) combinations of inducing or modifying factor and antagonist, in varying doses from less than 10 ng/ml up to about 10 µg/ml. After nine days of treatment, quantitative RT-PCR analyses are performed for expression of early, proliferative cartilage markers (e.g., collagen II, MIA), late, maturing cartilage marker (e.g., collagen X), and fibrous tissue marker (e.g., collagen I).

### Example 3

### Mouse bioassay for cartilage, bone, tendon and ligament induction

Similar to the Sampath and Reddi rat ectopic implant assay, a mouse ectopic implant assay, using outbred NMRI mice or inbred C3H mice, 2 months old, is performed (Sampath and Reddi, Proc. Natl. Acad. Sci. USA 80 (1983) 6591-6595; International Application No. WO 95/16035). The (a) BMP antagonist alone, (b) inducing or modifying factor alone, and (c) combinations of inducing or modifying factor and antagonist are lyophilized in the appropriate buffer, e.g., 0.1% trifluoroacetic acid for BMP proteins. For factor combinations buffers are chosen dependent on stability. Any suitable carrier may be used, e.g., collagen type I matrix, collagen-heparin mixture, gelatin capsules, hyaluronic acid or other functionally equivalent device, based on biocompatibility, biodegradability, stability and mechanical properties.

The implants are set intramuscularly into the hindlimb muscle of the mouse for 7 and 14 days. After 7 and 14 days the mice are sacrificed by cervical dislocation. The implants are isolated and processed using standard histological techniques (see Theory and Practice of Histological Techniques, ed. Bancroft and Stevens, Churchill Livingstone 1996). Paraffin (8 µm) or glycolmethacrylate sections (1 µm) are stained with Toluidine Blue, Alcian Blue, von Kossa, Movat or Hematoxylin/Eosin to visualize and quantitate the amount of tendon, ligament, cartilage and bone tissue induced in each implant. Positive (e.g., BMP-2, GDF-5) and negative (e.g., mock device) implant control groups are compared to experimental implants.

To assess the quality of cartilage and/or bone induced, gene expression can be studied by RNA in situ hybridization and quantitative RT-PCR for cartilage and bone markers as described above.

### Example 4

### Full thickness articular cartilage repair model

A full thickness articular cartilage defect model in the femoral-patellar joint of adult rabbits is used to assess the ability of the combinations of BMP antagonist, inducing or modifying factor and carrier to affect cartilage and bone repair. Adult rabbits are anesthetized and prepared for sterile surgery. An up to 4 x 4 mm defect through articular cartilage and into underlying subchondral bone is drilled into the patellar groove of the knee joint. The defect is either left empty, filled with the appropriate carrier, or filled with a carrier containing BMP antagonist and inducing or modifying factor or each factor alone. Animals are allowed to move freely for four weeks. After four weeks the animals are humanely euthanized and the articular cartilage/subchondral bone defect site is evaluated histologically for tissue architecture, quantity and quality of the repair.

### Example 5

### Partial thickness articular cartilage repair model

A partial thickness articular cartilage defect model in the femoral-patellar joint of adult rabbits is used to assess the ability of the combinations of BMP antagonist, inducing or modifying factor and carrier to affect cartilage and bone repair. Adult rabbits are anesthetized and prepared for sterile surgery. An up to 4 x 4 mm hole is drilled through articular cartilage into the patellar groove of the knee joint, leaving the underlying subchondral bone intact. The defect is either left empty, filled with the appropriate carrier, or filled with a carrier containing BMP antagonist and inducing or modifying factor or each factor alone. Animals are allowed to move freely for four weeks. After four weeks the animals are humanely euthanized and the articular cartilage defect site is evaluated histologically for tissue architecture, quantity and quality of the repair.

### Example 6

### Mouse bioassay for cartilage, bone, tendon and ligament induction using DNA

Similar to Sampath and Reddi rat ectopic implant assay, a mouse ectopic implant assay, using, e.g., outbred NMRI mice or inbred C3H mice, 2 months old is performed (Sampath and Reddi, Proc. Natl. Acad. Sci. USA 80 (1983) 6591-6595; International Application No. WO 95/16035). Expression vectors for (a) dorsalizing factor alone, (b) osteoinductive factor alone and (c) combinations of both are lyophilized in the appropriate buffer, e.g., TE-buffer (Fang et al., Proc. Natl. Acad. Sci. USA 93 (1996) 5753-5758). Any suitable carrier may be used, e.g., collagen type I matrix, collagen-heparin mixture, gelatin capsules, hyaluronic acid, or other functionally equivalent device, based on biocompatibility, biodegradability, stability and mechanical properties.

The implants are set intramuscular into the hindlimb muscle of the mouse for 7 and 14 days. After 7 and 14 days the mice are sacrificed by cervical dislocation. The implants are isolated and processed using standard histological techniques (see Theory and Practice of Histological Techniques, ed. Bancroft and Stevens, Churchill Livingstone, 1996). Paraffin (8 µm) or glycolmethacrylate sections (1 µm) are stained with Toluidine Blue, Alcian Blue, von Kossa, Movat or Hematoxylin/Eosin to visualize and quantitate the amount of tendon, ligament, cartilage or bone tissue induced in each implant. Positive (e.g., BMP-2, GDF-5) and negative (e.g., mock device) implant control groups are compared to experimental implants. To assess the quality of cartilage and/or bone induced, gene expression can be studied by RNA in situ hybridization and quantitative RT-PCR for cartilage and bone markers as described above.

### List of References

d'Angelo and Pacifici, J. Bone and Min. Res. 12 (1997) 1368-1377
Fainsod et al., EMBO 13 (1994) 5015-5025
Fainsod et al., Mechanisms of Development 63 (1997) 39-50
Fang et al., Proc. Natl. Acad. Sci. USA 93 (1996) 5753-5758
Heath and Margari, Biotechnology and Bioengineering 50 (1996) 430-437
Hemmati-Brivanlou et al., Cell 79 (1994) 169-179
Kinto et al., FEBS Letters 404 (1997) 319-323
Lamb et al., Science 262 (1993) 713-718
Nakamura et al., Science 247 (1990) 836-838
Piccollo et al., Cell 86 (1996) 12141-12145
Piccollo et al., Cell 86 (1996) 589-598
Re'em-Kalma et al., Proc. Natl. Acad. Sci. USA 92 (1995) 12141-12145
Reddi, A.H., Cytokine & Growth Factor Reviews 8 (1997) 11-20
Ruiz i Altaba, Essential Developmental Biology, A Practical Approach, IRL Press, 1993, IRL
Press, pp. 147-152
Sampath and Reddi, Proc. Natl. Acad. Sci. USA 80 (1983) 6591-6595
Sasai et al., Cell 79 (1994) 779-790
Sasai et al., Nature 376 (1995) 333-336
Steele and Copping, Postimplantation Mammalian Embryos, A Practical Approach, IRL
Press 1990, 229-232
Theory and Practice of Histological Techniques, ed. Bancroft and Stevens, Churchill
Livingstone 1996
U.S. Patent 5,486,359
U.S. Patent 5,670,481
U.S. Patent 5,679,783
WO95/16035
Zimmermann et al., Cell 86 (1996) 599-606

## Claims

1. A pharmaceutical composition containing a composition of an osteoinductive protein and a dorsalizing factor at a ratio of 10:1 to 1:10.

2. A pharmaceutical composition as claimed in claim 1, wherein the osteoinductive protein is BMP-2 or a hedgehog protein.

3. A pharmaceutical composition as claimed in claim 1 or 2, wherein the dorsalizing factor is noggin, chordin, or follistatin.

4. A pharmaceutical composition as claimed in claims 1 to 3, wherein the composition includes a biocompatible matrix.

5. A pharmaceutical composition as claimed in claims 1 to 4, wherein the biocompatible matrix is hyaluronic acid or alginate.

6. A method for manufacturing a pharmaceutical composition containing an osteoinductive protein and a dorsalizing factor at a ratio of 10:1 to 1:10, by combining said protein and said dorsalizing factor.

7. A method as claimed in claim 6, wherein the osteoinductive factor is BMP-2 or a hedgehog protein.

8. A method as claimed in claim 6 or 7, wherein the dorsalizing factor is noggin, chordin, or follistatin.

9. A method as claimed in claims 6 to 8, wherein the osteoinductive protein and the dorsalizing factor are combined with a biocompatible matrix.

10. A method as claimed in claim 9, wherein the biocompatible matrix is hyaluronic acid or alginate.

11. The use of a combination of an osteoinductive protein and a dorsalizing factor, for the treatment of a patient in need of cartilage repair.

12. A pharmaceutical composition containing an expression vector for an osteoinductive protein and a dorsalizing factor or a combination of a vector for the expression of an osteoinductive protein with a vector capable of expression of a dorsalizing factor.

13. A method for manufacturing a pharmaceutical composition containing an expression vector capable of expression of an osteoinductive protein and a dorsalizing factor, or a vector capable of expression of an osteoinductive protein and a vector capable of expression of a dorsalizing factor.
